# EUROPEAN PATENT APPLICATION

(11) **EP 4 001 261 A1**
(43) Date of publication of application: **25.05.2022**
(21) Application number: 20383019.5
(22) Date of filing: 23.11.2020
(51) Int. Cl.: C07C 231/14, C07C 231/12, C07C 237/46

(54) **PROCEDURE FOR OBTAINING HIGH PURITY IOHEXOL**

(71) Applicant: Justesa Imagen S.A.U, 28823 Coslada (ES)
(72) Inventor: ALONSO SILVA, Ignacio, 28823 Coslada, Madrid (ES); CERECEDA PÉREZ, Juan Andrés, 28823 Coslada, Madrid (ES); MARTÍN MARTÍN, Felix Ramón, 28823 Coslada, Madrid (ES); CHICHARRO MARTÍN, Roberto, 28823 Coslada, Madrid (ES); GARCÍA RAMOS, Álvaro, 28823 Coslada, Madrid (ES); GARCÍA SALADO, Irene, 28823 Coslada, Madrid (ES)
(74) Representative: Elzaburu S.L.P.

(57) **Abstract**

The present invention refers to a procedure to obtain iohexol from TAIPA in which the formation of acetyl-TAIPA occurs without the use of alcoholic solvents and its subsequent alkylation with (±)-1-chloro-2,3-propanediol (CPD) to obtain crude iohexol using low toxicity solvents and under reaction conditions which allow to minimize the formation of impurities, increase the overall yield and simplify operation.

## Description

### FIELD OF THE INVENTION

The present invention relates to an industrial process of manufacturing the X-ray contrast agent iohexol (5-(*N*-2,3-dihydroxypropylacetamide)-2,4,6-triyodo-*N*,*N*'-bis(2,3-dihydroxypropyl)isophthalamide) from TAIPA (*N*,*N'*-bis(2,3-dihydroxypropyl)-5-amino-2,4,6-triyodoisoftalamide). Therefore, the present invention is included in the field of pharmaceutical chemistry and pharmacology.

### BACKGROUND OF THE INVENTION

lohexol is a non-ionic, monomeric, tri-iodinated, water-soluble radiological contrast agent. The product is marketed under different brand names such as NITIGRAF^{®} (Juste S.A.-Q.F.), OMNIPAQUE^{®} (GE Healthcare), etc.

lohexol is commonly used as an X-ray contrast media. lohexol can be used in several radiographic procedures and is usually administered intravenously. It is used for angiography, exploration of the urinary system, the spinal cord, the lymphatic system, etc. The advantages of iohexol are its low viscosity, osmolality, toxicity and high tolerance.

There are a large number of methods described for the manufacture of iohexol; some examples are listed in the following patents: US4250113, ES8602419, ES8505940, US5191119 and ES2017277. However, several of the procedures described in these patents are currently not suitable for large-scale production, since they require numerous synthetic steps, the use of reagents with high environmental impact (EP0118347) and expensive purification processes that use, in most of the cases, among others, a treatment with organic solvents (n-butanol, isopropanol) in multiple crystallizations. This implies low yields and the risk of administering intravenously a product that contains traces of potentially toxic organic solvents. Therefore, there is a clear need to improve and optimize current iohexol production processes.

### DESCRIPTION OF THE INVENTION

The present invention is directed to solving the disadvantages of the present procedures for obtaining iohexol, describing a process which includes a synthesis with several optimized stages and various methods of purification and isolation, which results in a product of high purity. The procedure described below presents the advantages of using reaction conditions that minimize the formation of impurities, increase overall yield and simplify operations.

The present invention describes the methodology to obtain iohexol. The synthetic route is described in the following scheme:

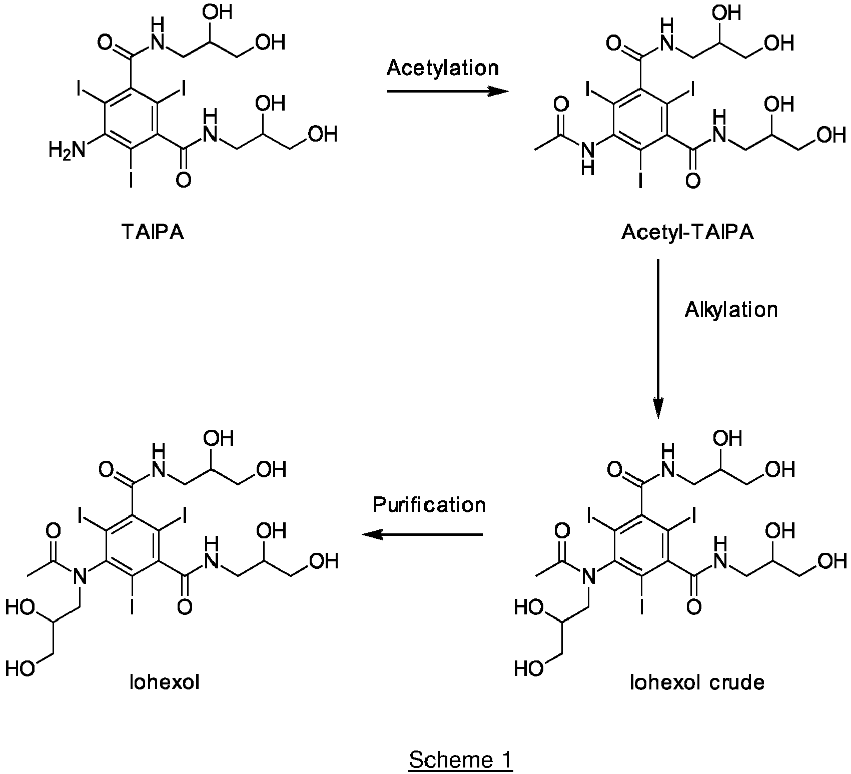

In a first aspect, the present invention refers to a procedure to obtain high purity 5-(*N*-2,3-dihydroxypropylacetamido)-2,4,6-triyodo-*N*,*N'*-bis(2,3-dihydroxypropyl) isophthalamide (iohexol) which comprises the following steps:
(a) obtention of 5-(acetamide)-*N*,*N'*-bis(2,3-dihydroxypropyl)-2,4,6-triyodoisophthalamide (acetyl-TAIPA) by a per-acetylation reaction in an media free of alcoholic solvents which comprises:
   a1) reaction of *N*,*N'*-bis(2,3-dihydroxypropyl)-5-amino-2,4,6-triyodoisoftalamide (TAIPA) with acetic anhydride in glacial acetic acid, in the presence of a catalyst which is an acid, preferably sulfuric acid or p-toluensulfonic acid;
   a2) hydrolysis in basic medium of the obtained per-acetyl-TAIPA in step a1) by adding the reaction crude to a strong base solution of an alkaline or alkaline-earth element, preferably sodium hydroxide, maintaining the pH constant between 10-12 and the temperature between 40-80 ºC, preferably at pH 11 and 50 ºC;
   a3) precipitation of acetyl-TAIPA by adding the acetyl-TAIPA solution obtained in step a2) onto a solution of an acid, preferably acetic acid, hydrochloric acid or sulfuric acid, keeping the pH lower than 6 and the temperature between 40-80 ºC, preferably at pH 1 and 50 ºC;
(b) obtention of crude iohexol by alkylation reaction of acetyl-TAIPA obtained in the previous step (a) with (±)-1-chloro-2,3-propanediol (CPD) in the presence of a solvent selected from an alcohol, an alkyl-alcohol or a polyalcohol, preferably, 2-ethoxyethanol or propylene glycol;
(c) purification of crude iohexol obtained in the previous step (b); and
(d) isolation of the iohexol obtained in the previous step (c) by crystallization and/or spray drying.

It is noteworthy that step (a) of the procedure to obtain acetyl-TAIPA from TAIPA does not use alcoholic solvents such as methanol. This step consists of two reactions: a per-acetylation reaction followed by a hydrolysis reaction in basic medium and a precipitation in acidic medium, as shown in the following scheme:

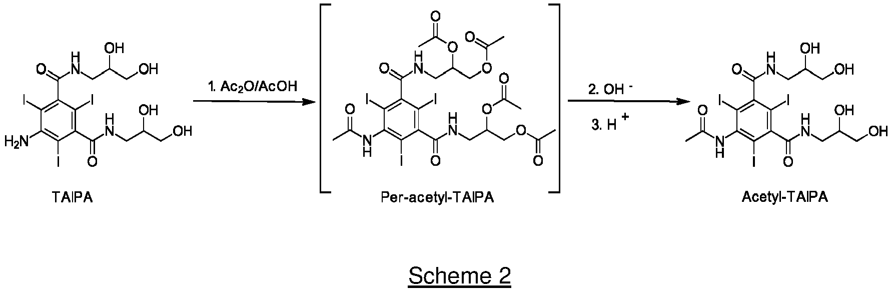

In one way of proceeding with said step (a), a solution of TAIPA is prepared in a mixture of glacial acetic acid and an acid catalyst. Acetic anhydride is then added. In a preferred embodiment, said 'acid catalyst' is sulfuric acid or p-toluensulfonic acid.

The per-acetyl-TAIPA initially obtained in this step, precipitates significantly as it is formed, making difficult their subsequent handling. In the known iohexol synthesis procedures, to avoid this precipitation, the product is dissolved in methanol, a highly toxic product that needs to be removed from the reaction medium. On the contrary, in the procedure of the invention, the inventors have determined that intermediate pH boosts the precipitation and, therefore, the addition of the reaction crude of step (a1) directly onto a basic pH solution prevents this precipitation.

Specifically, the reaction is heated for a certain time until the formation of per-acetyl-TAIPA is completed. Once the reaction is completed, the hydrolysis reaction takes place. The reaction crude is added to a solution at basic pH containing a strong base of an alkaline or alkaline-earth element, preferably potassium, sodium or lithium hydroxide, and more preferably sodium hydroxide, maintaining the pH and temperature, and thus forming the acetyl-TAIPA.

With the procedure described in the present invention an acetyl-TAIPA which presents an improvement in the chromatographic profile is obtained, as well as an absence of highly toxic residual solvents such as methanol. This behavior can be observed in the following table:

**Table 1**

| | Acetyl-TAIPA purity (HPLC) | Methanol (ppm-GC) |
|---|---|---|
| Methanol procedure | >98% | 200-6000 |
| Acetic acid/acetic anhydride procedure | >99.5% | n.d. |

In a preferred embodiment, the pH of hydrolysis is 11.

In a preferred embodiment, the temperature of the hydrolysis is 50 ºC.

Then, the precipitation is carried out, and for this purpose acetyl-TAIPA solution (in salt form) is added to a solution at acidic pH, keeping the pH and temperature constant.

The pH is kept constant and lower than 6 by adding an acid that is selected from hydrochloric acid, sulfuric acid, acetic acid, preferably hydrochloric acid or acetic acid. In a preferred embodiment, the pH of the precipitation is 1. The temperature of the precipitation is comprised between 40 and 80 ºC. In a preferred embodiment, the temperature is 50 ºC.

Another problem observed by following the usual procedures to obtain iohexol is that acetyl-TAIPA is obtained with a very fine particle size, which causes great difficulty in the isolation process, requiring large filtration surfaces and process time, also requires a drying and grinding stage, which further increases the difficulty of the process. In the present invention, this problem is avoided by means of a decantation and subsequent dissolution of the reaction crude obtained in (a3). This dissolution is carried out with an alcohol, an alkyl-alcohol or a polyalcohol, preferably, 2-ethoxyethanol or propylene glycol, which are the solvents in which the reaction between the acetyl-TAIPA and the CPD will take place. The reaction is shown below:

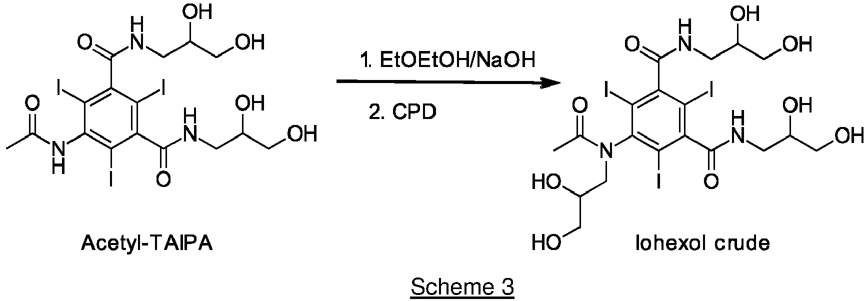

By using 2-ethoxyethanol or propylene glycol as solvents, the reaction is achieved in shorter time and the formation of *O*-alkylated impurities is minimized. This can be observed by comparing the chromatographic profiles related to the alkylation step using various solvents described in the above-mentioned patents and the solvents claimed in the present invention, as can be seen in the following table

**Table 2**

| Solvent (reaction time) | Cycled* (%) | Acetyl-TAIPA** (%) | IHX (%) | TAIPA*** (%) | *O-alkylated* (%) | *O-Acetylated* -(%) |
|---|---|---|---|---|---|---|
| Methanol (22 hours) | 0,3 | 1,8 | 93,0 | 1,3 | 3,2 | 0,4 |
| Methoxyethanol (24 hours) | 0,3 | 1,8 | 94,9 | 1,3 | 1,5 | 0,2 |
| Propylene Glycol (4 hours) | n.d.. | 0,2 | 97,5 | 1,5 | 0,7 | 0,1 |
| Ethoxyethanol (18 hours) | 0,1 | 0,6 | 97,2 | 0,9 | 1,1 | 0,1 |

| | | | | | | |
|---|---|---|---|---|---|---|
| * - Impurity I according to the European Pharmacopoeia; ** - Impurity A according to the European Pharmacopoeia; *** - Impurity J according to the European Pharmacopoeia | | | | | | |

In general, an acetyl-TAIPA solution is prepared at basic pH in a solvent such as alcohols, alkyl-alcohols or polyalcohols. Then the CPD is added. The reaction is heated for a certain time until the formation of iohexol is completed.

In a preferred embodiment, the acetyl-TAIPA obtained in step (a) is isolated and dried prior to step (b).

In this case where the acetyl-TAIPA is previously isolated, the amount of solvent in step (b) is comprised between 0.5 and 7 mL per g of acetyl-TAIPA. In a more preferred embodiment, the amount of solvent in step (b) is 1 to 3 mL per g of acetyl-TAIPA. In an even more preferred embodiment the amount of solvent is 1.5 to 2 mL per g of acetyl-TAIPA.

In another preferred embodiment, before proceeding with step (b), the acetyl-TAIPA obtained in step (a) is not isolated, and the supernatant is decanted at least once and the solid is washed with water at least once prior to step (b).

The process of per-acetylation, hydrolysis and precipitation is carried out in the same way as in step (a) and the solid formed is purified, without isolation, by processes of decantation and washing with water.

In a preferred embodiment if acetyl-TAIPA has not been isolated, the washings are comprised between 4 and 10 volumes and the number of decantings is comprised between 2 and 6. In a more preferred embodiment, the washings are with 7 volumes and the number of decantings are 3.

In a preferred embodiment if acetyl-TAIPA has not been isolated, a change of solvent is carried out, going from having a suspension of acetyl-TAIPA in water to obtaining a solution of acetyl-TAIPA in a solvent such as 2-ethoxyethanol, or propylene glycol among others. This process is carried out by distillation and addition of fresh solvent.

In a preferred embodiment if acetyl-TAIPA has not been isolated, the amount of solvent used is comprised between 0.5 and 7 mL per g of acetyl-TAIPA. In a more preferred embodiment, the amount of solvent is between 1 and 4 mL per g of acetyl-TAIPA.

In a preferred embodiment, the reaction temperature in step (b) is comprised between 10 and 60 ºC. In a more preferred embodiment, the temperature of the reaction is comprised between 20 and 35 ºC.

In another preferred embodiment, the amount of CPD in step (b) is comprised between 1 and 3 moles per mole of acetyl-TAIPA. In a more preferred embodiment, the amount of CPD is comprised between 1.3 and 2 moles per mole of acetyl-TAIPA.

In another preferred embodiment, the amount of CPD in step (b) can be added in one fraction or in several. In a more preferred embodiment, the amount of CPD is added in two fractions.

The procedure of the invention also comprises a step of purification of crude iohexol (step c).

The reaction crude from the alkylation step, which contains iohexol, related compounds and inorganic salts, is purified by treatment with granular carbon column, powdered carbon, carbon cartridges, filtration processes, ion exchange resins, macroporous resins, or nanofiltration techniques, electrodialysis, or a combination of the above using different types of membranes.

In a preferred embodiment, the purification is carried out by a filtration process followed by a treatment with an anion exchange resin, a treatment with a cation exchange resin and a treatment with a granular carbon column.

In a preferred embodiment, the amount of carbon in the column is comprised between 5 and 50% of the amount of iohexol to be purified. In a more preferred embodiment, the amount of carbon in the column is 20% of the amount of iohexol to be purified.

In a preferred embodiment, the amount of exchange resins is comprised between 0.05 and 0.5 g of resin per g of iohexol to be purified. In a more preferred embodiment, the amount of ion exchange resins is 0.20 g of resin per g of iohexol to be purified.

The deionized iohexol is then crystallized using solvents such alcohols or alkyl-alcohols and then depyrogenated using depyrogenizing agents or techniques. In a more preferred embodiment, these solvents for crystallization are ethanol or 2-ethoxyethanol.

In a comparison of various solvents for the crystallization step described in the present invention, an improvement in the chromatographic profile can be observed when the above-mentioned solvents (ethanol or 2-ethoxyethanol) are used, requiring a single crystallization to achieve a product which meets the specifications. This can be observed in the following table:

**Table 3**

| Crystallization solvent | Cycled (%) | Ac. Taipa (%) | IHX (%) | TAIPA (%) | *O*-alkylated (%) | O-Acetylated (%) | CPD (ppm) |
|---|---|---|---|---|---|---|---|
| **lohexol purified by resins and qranular carbon** | | | | | | | |
| | 0,2 | 1,1 | 94,7 | 0,2 | 1,2 | 0,1 | 48 |

| **lohexol purified by crystallization** | | | | | | | |
|---|---|---|---|---|---|---|---|
| *n*-Butanol | 0,2 | 1,1 | 97,1 | 0,2 | 1,1 | 0,1 | N.A. |
| Isopropanol | 0,1 | 1,0 | 97,5 | 0,2 | 1,0 | 0,1 | N.A. |
| 2-Ethoxvethanol | N.A. | N.A. | 99,6 | N.A. | 0,4 | N.A. | N.A. |
| Ethanol | N.A. | N.A. | 99,8 | N.A. | 0,2 | N.A. | N.A. |

In a preferred embodiment, the amount of solvent in the crystallization is comprised between 1 and 5 volumes, and the temperature comprised between 40 and 100 ºC. In a more preferred embodiment, the amount of solvent in the crystallization is comprised between 2 and 4 volumes, and the temperature is 80 ºC.

In a preferred embodiment, the product is depyrogenated by treatment with aluminum oxide or ultrafiltration techniques.

The procedure of the invention also comprises an iohexol isolation step (step d).

In a preferred embodiment, the isolation of purified iohexol is carried out by spray drying or crystallization with organic solvents or a combination of both techniques.

In a more preferred embodiment, purified iohexol is spray dried.

In another preferred embodiment, purified iohexol is isolated by crystallization with organic solvents such as alcohols or alkyl-alcohols and more preferably ethanol.
In a preferred embodiment, the amount of solvent in the crystallization is comprised between 1 and 5 volumes, and at a temperature comprised between 40 and 90 ºC.

In a more preferred embodiment, the amount of solvent in the crystallization is comprised between 2 and 4 volumes, and the temperature is 80 ºC.

In a preferred embodiment the isolated product from a crystallization is dissolved in water and isolated and dried by spray drying.

### EXAMPLES OF THE INVENTION

### Example 1. Preparation of acetyl-TAIPA (step a) from TAIPA,

A suspension of TAIPA (820 kg) in glacial acetic acid (820 L) is prepared and concentrated sulfuric acid (4.20 kg) is added. The mixture is heated to 75 ºC and acetic anhydride (820 L) is added during approximately 90 min maintaining the temperature. After the addition, the mixture is kept at the same temperature for 3 h. Then it is cooled down to 45 ºC and the acetic anhydride/acetic acid mixture (1200 L) is distilled under vacuum. After that, 500 L of water is added to the distillation residue. This solution is added to a previously prepared solution of NaOH 50% (w/w) (700 kg) in water (1500 L) at 50 ºC, maintaining the pH and temperature for 2 h. This solution is slowly added onto a previously prepared solution of hydrochloric acid (510 L) in water (2000 L) at 50 ºC, maintaining the pH and temperature, obtaining a precipitate. The resulting suspension is cooled down to room temperature, filtered, washed with water (400 L) and dried (823 kg, 95% yield).
FT-IR (cm-1): 3232, 3209, 1654, 1629, 1550, 1508, 1421, 1350, 1273, 1053

### Example 2. Preparation of crude iohexol from acetyl-TAIPA,

### 2.1 Using 2-Ethoxyethanol as solvent

The previously obtained acetyl-TAIPA (500 kg) is added onto a solution of 2-ethoxyethanol (900 L) and NaOH 50% (w/w) (80 kg). The mixture is heated to 50 ºC until complete dissolution, then cooled down to 23 ºC and a first fraction of CPD (96.2 kg) is added and after 2 hours, a second fraction of 15 kg is added. The mixture is stirred for 16 hours. Then hydrochloric acid (10 L) is added up to pH 5.5 and the salts formed are filtered. The mixture is distilled under vacuum (700 L of 2-ethoxyethanol) and 2000 L of water are added to the distillation residue, resulting in a solution of crude iohexol for subsequent purification (539 kg, 98% yield).

### 2.2 Using propylene glycol as solvent

Acetyl-TAIPA (500 kg) is added onto a propylene glycol solution (900 L) and NaOH 50% (w/w) (80 kg), . The mixture is heated to 50 ºC until complete dissolution, then it is cooled to 23 ºC and a first fraction of CPD (96.2 kg) is added and after 2 hours a second fraction of 15 kg is added. The mixture is stirred for 2 hours. Then hydrochloric acid (10 L) is added up to pH 5.5 and the salts formed are filtered. The mixture is distilled under vacuum (700 L of propylene glycol) and 2000 L of water are added to the distillation residue, resulting in a solution of crude iohexol for subsequent purification (533 kg, 97% yield).

### Example 3. Preparation of crude iohexol using non-isolated acetyl-TAIPA as an intermediate

A suspension of TAIPA (6 kg) in glacial acetic acid (6 L) is prepared. Concentrated sulfuric acid (16 mL) is then added. The mixture is heated to 75 ºC and acetic anhydride (6 L) is added for approximately 90 min while maintaining the temperature. After the addition, the mixture is heated to 75 ºC for 3 h. Then it is cooled to 45 ºC and the mixture acetic anhydride/acetic acid (6.70 L) is distilled under vacuum and 3 L of water are added to the distillation residue. This solution is slowly added onto a previously prepared of NaOH 25% (w/v) (11 L) in water (6 L) at 50 ºC maintaining the pH and temperature for 2 h. This solution is slowly added onto a previously prepared solution of 80% (13 L) acetic acid in water (2.5 L) at 50 ºC maintaining the pH and temperature, forming a precipitate. The resulting suspension is cooled down to room temperature, decanted and washed with water (40 L x 3). It is distilled under vacuum (5.25 L) and the residue is dissolved in 2-ethoxyethanol (16 L) with NaOH 50% (w/w) (1 kg) at 50 º. This mixture is stirred until complete dissolution, then it is cooled to 23 ºC and CPD is added (1.25 kg). The mixture is stirred for 18 hours. After that, hydrochloric acid (0.25 L) is added up to pH 4.5 and the salts formed are filtered. 2-Ethoxyethanol (12 L) is distilled under vacuum and 18 L of water are added to the distillation residue, resulting in a solution of crude iohexol for subsequent purification (6.70 kg, 95% yield from TAIPA).

### Example 4. Purification of Crude iohexol (step c).

An aqueous solution of crude iohexol is sequentially passed through a train of ion exchange resin columns (IRA-67 and IR-120H) and a granular carbon column for 24 h at a flow rate of 1800 L/h. Then, water is distilled under vacuum (900 L) and 2000 L of 2-ethoxyethanol are added to the distillation residue. The mixture is then heated to 80 ºC for 8 hours, forming a precipitate. The resulting suspension is cooled down to room temperature, centrifuged and washed with 2-ethoxyethanol (110 L), obtaining purified wet iohexol for subsequent isolation step (620 kg, 86% yield).

### Example 5. Isolation of iohexol by crystallization (step d),

Alumina (22.5 kg) is added to a solution of the solid from step c) in water (700 L). The mixture is stirred for 1 hour, filtered, and to the resulting solution, ethanol (1000 L) is added. The mixture is heated to reflux for 8 hours, forming a precipitate. The resulting suspension is cooled to room temperature, filtered, washed with ethanol (800 L), and dried to obtain a product that meets the required specifications according to the pharmacopoeias of the United States and Europe (468 kg, 85% yield).
FT-IR (cm-1): 3290, 3257, 3234, 1631, 1550, 1394, 1373, 1255, 1109, 1033

### Example 6. Isolation of iohexol by spray drying (step d),

A solution of the solid from step c) in water (700 L) is subjected to a depyrogenating ultrafiltration and then isolated by spray drying. A product that meets the specifications required by the pharmacopoeias of the United States and Europe is obtained (475 kg, 86% yield).

## Claims

1. A procedure to obtain 5-(*N*-2,3-dihydroxypropylacetamido)-2,4,6-triyodo-*N*,*N'-*bis(2,3-dihydroxypropyl)isophthalamide (iohexol) of high purity that comprises the following steps:
(a) obtention of 5-(acetamide)-*N*,*N'*-bis(2,3-dihydroxypropyl)-2,4,6-triyodoisophthalamide (acetyl-TAIPA) by a per-acetylation reaction in a medium free of alcoholic solvents which comprises:
a1) reaction of *N*,*N*-bis(2,3-dihydroxypropyl)-5-amino-2,4,6-triyodoisoftalamide (TAIPA) with acetic anhydride in glacial acetic acid, in the presence of a catalyst which is an acid, preferably sulfuric acid or *p*-toluensulfonic acid;
a2) hydrolysis in basic medium of the per-acetyl-TAIPA obtained in step a1) by adding the reaction crude to a strong base solution of an alkaline or alkaline-earth element, preferably sodium hydroxide, keeping constant the pH between 10-12 and the temperature between 40-80 ºC, preferably at pH 11 and temperature 50 ºC;
a3) precipitation of acetyl-TAIPA by adding the acetyl-TAIPA solution obtained in step a2) onto a solution of an acid, preferably acetic acid, hydrochloric acid or sulfuric acid, keeping the pH lower than 6 and the temperature between 40-80 ºC, preferably at pH 1 and 50 ºC;
(b) obtention of crude iohexol by an alkylation reaction of the acetyl-TAIPA obtained in the previous step (a) with (±)-1-chloro-2,3-propanediol (CPD) in the presence of a solvent selected from an alcohol, an alkyl-alcohol or a polyalcohol, preferably, 2-ethoxyethanol or propylene glycol;
c) purification of the crude iohexol obtained in the previous step; and
d) isolation of the final product by crystallization and/or spray-drying

2. The procedure according to claim 1, where the acetyl-TAIPA obtained in step (a) is isolated and dried before step (b).

3. The procedure according to claim 1, where the acetyl-TAIPA obtained in step (a) is not isolated and is subjected to at least one decantation and at least one washing with water before step (b).

4. The procedure according to any of the claims 1 to 3, where in step (b), the amount of solvent is comprised between 0.5 to 7 mL per g of acetyl-TAIPA,

5. The procedure according to any of claims 1 to 4, where step (b) is carried out in a strong basic medium, preferably sodium hydroxide or sodium methoxide.

6. The procedure according to any of the claims 1 to 5, where the amount of CPD used in step (b) is comprised between 1 to 3 moles per mole of acetyl-TAIPA, preferably 1.3-2 moles per mol.

7. The procedure according to any of the claims 1 to 6, where the CPD is added at once or in several fractions, preferably in two fractions.

8. The procedure according to any of the claims 1 to 7, where the temperature of the reaction of step (b) is comprised between 10-60 ºC, preferably at 20-35 ºC.

9. The procedure according to any of the claims 1 to 8, in which the purification of crude iohexol from step (c) is performed by a treatment with granular carbon column, powdered carbon, carbon cartridges, filtration processes, ion exchange resins, macroporous resins, or nanofiltration techniques, electrodialysis, or a combination of the above.

10. The procedure according to claim 9, in which the purification is carried out by a filtration process followed by a treatment with an anion exchange resin, a treatment with a cation exchange resin and a treatment with a granular carbon column.

11. The procedure according to any of the claims 9 or 10 in which a crystallization is subsequently carried out with alcohols or alkyl-alcohols, preferably ethanol or 2-ethoxyethanol, and a subsequent depyrogenation by treatment with alumina or ultrafiltration.

12. The procedure according to any of the claims 1 to 11, where the isolation of iohexol from step (d) is carried out by crystallization with organic solvents, preferably ethanol or 2-ethoxyethanol.

13. The procedure according to claim 12, in which 1-5 volumes of organic solvent are used in the crystallization, preferably 2-4 volumes.

14. The procedure according to any of the claims 12 or 13, where the crystallization is carried out at a temperature comprised between 40-90 ºC, preferably at 80 ºC

15. The procedure according to any of the claims 1 to 11, where the isolation of iohexol from step (d) is carried out by spray drying.
